# EUROPEAN PATENT APPLICATION

(11) **EP 4 759 304 A1**
(43) Date of publication of application: **17.06.2026**
(21) Application number: 24218800.1
(22) Date of filing: 10.12.2024
(51) Int. Cl.: A61K 9/16, A61K 31/4545

(54) **PELLET COMPOSITIONS COMPRISING APIXABAN AND METHODS FOR THE PRODUCTION THEREOF**

(71) Applicant: Laboratoires S.M.B., 1080 Bruxelles (BE)
(72) Inventor: BAUDIER, Philippe, Casablanca (MA); SEBTI, Thami, 1090 Brussels (BE); MERLOS, Romain, 7070 Mignault (BE)
(74) Representative: Kirkpatrick

(57) **Abstract**

The invention relates to dry compositions in the form of pellets suitable for facilitating administration of apixaban. Furthermore, the invention relates to method for producing pellet compositions comprising apixaban.

## Description

### FIELD OF THE INVENTION

The invention relates to dry pellet compositions comprising apixaban and methods for the production of said compositions.

### BACKGROUND OF THE INVENTION

Apixaban is an anticoagulant that inhibits factor Xa, an essential enzyme in the blood coagulation cascade. Apixaban has the structure:

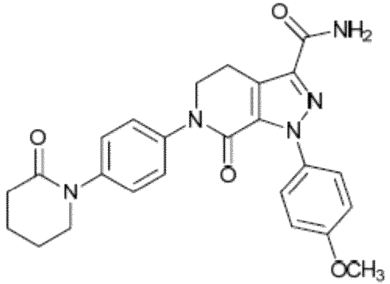

The chemical name of Apixaban (IUPAC) is 1-(4-Methoxyphenyl)-7-oxo-6-[4-(2-oxopiperidin-1-yl)phenyl]-4,5,6,7-tetrahydro-1H-pyrazolo[3,4-c]pyridine-3-carboxamide.

A process for the synthesis of apixaban is described in U.S. patent 6,967,208.

Apixaban is used for the prevention and treatment of thromboembolic disorders, including:
- prevention of venous thromboembolic events (VTE) in adult patients who have undergone elective hip or knee replacement surgery,
- prevention of stroke and systemic embolism in adult patients with non-valvular atrial fibrillation (NVAF), and
- treatment of deep vein thrombosis (DVT) and pulmonary embolism (PE) and prevention of recurrent DVT and PE in adults.

EP3017811 describes tablets comprising crystalline apixaban particles produced by a dry granulation process. EP3017811 describes that formulations made using a wet granulation process resulted in less optimal exposures in bioavailability studies.

EP3781132 describes sugar spheres coated with a solution containing apixaban suitable for administering small doses of apixaban to children.

### SUMMARY OF THE INVENTION

The present invention relates to dry pellet compositions comprising apixaban that facilitate administration of apixaban to a patient.

Apixaban is currently mostly provided in the form of a tablet. While effective and convenient in many situations, some patients have difficulties swallowing tablets. For such patients, the tablet is often crushed and dispersed in water or food for oral administration or administration via a nasogastric tube. However, crushing of tablets may be cumbersome and lead to loss of material. Furthermore, upon administration via the nasogastric route, large or irregularly shaped fragments may not flow smoothly through the tubing or even block the tube.

The present invention provides a dry composition comprising apixaban which solves the above-mentioned issues. The composition is in the form of pellets, which may be provided in a dose-unit container such as a capsule. Upon use, the dose-unit container may be opened by applying light pressure and the pellets will flow smoothly into a liquid medium or a non liquid food substance or for oral and/or nasogastric administration.

Thus, the pellets of the invention provide a composition comprising apixaban having good flow properties.

The invention also provides a robust convenient wet manufacturing process for producing the composition, resulting in a highly homogeneous product. Surprisingly, it was found that the product resulting from the wet process has excellent bioavailability.

Accordingly, in a first main aspect, the invention provides a dry composition in the form of pellets suitable for facilitating administration of apixaban, comprising:
(i) crystalline apixaban particles having a D90 of less than 50 micrometers, as measured by laser light scattering,
(ii) lactose and microcrystalline cellulose in a % ratio of between 99 to 1 and 90 to 10, or
   mannitol and microcrystalline cellulose in a % ratio of between 99 to 1 and 90 to 10,
(iii) a further binder which is not microcrystalline cellulose, and
(iv) a surfactant.

In a further main aspect, the invention provides a dose-unit container comprising a dry pellet composition according the invention.

In a further main aspect, the invention provides a method for the preparation of a dry pellet composition comprising apixaban, comprising the steps of:
(i) Mixing excipients and apixaban simultaneously or sequentially,
(ii) Adding an amount of an aqueous liquid sufficient to obtain a paste,
(iii) Extruding, spheronising and drying to obtain dry pellets, optionally comprising a further step of selecting pellets having a size within a pre-determined size range.

In a further main aspect, the invention provides a dry pellet composition obtainable by said method.

### BRIEF DESCRIPTION OF THE FIGURES

Figure 1: Influence of fillers on dissolution profiles of apixaban pellets in 900mL pH 1.2 (HCl 0.1N) at 37°C using paddle apparatus at 75 rpm
Figure 2: Influence of binder on dissolution profiles of apixaban pellets in 900mL pH 1.2 (HCl 0.1N) at 37°C using paddle apparatus at 75 rpm.
Figure 3: Influence of surfactant on dissolution profiles of apixaban pellets in 900mL pH 1.2 (HCl 0.1N) at 37°C using paddle apparatus at 75 rpm
Figure 4: Influence of apixaban particle size on dissolution profiles of apixaban pellets in 900mL pH 1.2 (HCl 0.1N) at 37°C using paddle apparatus at 75 rpm
Figure 5: Mean plasma concentration profiles of APX003 and Eliquis^{®}.

### DETAILED DESCRIPTION OF THE INVENTION

As described above, in a main first aspect, the invention relates to a dry composition in the form of pellets suitable for facilitating administration of apixaban, comprising:
(i) crystalline apixaban particles having a D90 of less than 50 micrometers, as measured by laser light scattering,
(ii) lactose and microcrystalline cellulose in a % ratio of between 99 to 1 and 90 to 10, or
   mannitol and microcrystalline cellulose in a % ratio of between 99 to 1 and 90 to 10,
(iii) a further binder which is not microcrystalline cellulose, and
(iv) a surfactant.

Similarly, the invention relates to a dry composition in the form of pellet, said comprising:
(i) crystalline apixaban particles having a D90 of less than 50 micrometers, as measured by laser light scattering,
(ii) lactose and microcrystalline cellulose in a % ratio of between 99 to 1 and 90 to 10, or
   mannitol and microcrystalline cellulose in a % ratio of between 99 to 1 and 90 to 10,
(iii) a further binder which is not microcrystalline cellulose, and
(iv) a surfactant.

When used herein, the term "pellets" refers to uniform, cylindrical or spherical particles. Pellets have a defined, compact shape and are typically formed by processes like extrusion, compaction, or pelletization, where material is compressed or shaped into a uniform form. The term "pellets" when used herein does not encompass granules, which are irregular, coarse particles that are not uniform in shape. Granules may look like small, rough chunks or grains and are often created by crushing or grinding larger materials or through granulation processes like sprayer layering, spray drying or fluidized bed techniques.

In one embodiment of the dry pellet composition of the invention, wherein the pellets have a diameter of between 200 micrometers and 2000 micrometers, such as between 800 micrometers and 1600 micrometers.

The dry pellet composition of the invention comprises crystalline apixaban particles. The apixaban form may be Form H2-2, or, more preferably, Form N-1.

The composition of the invention may comprise between 1 and 5% (w/w) of crystalline apixaban particles, such as between 1.5 and 4.5%, between 2 and 4%, between 2.5 and 3.5%, between 3 and 3.5% or between 3 and 3.2%, such as 3.1% of crystalline apixaban particles.

The crystalline apixaban particles used in the composition of the invention have a D90 of less than 50 micrometers, as measured by laser light scattering, such as a D90 of less than 40 micrometers, for example less than 35 micrometers, such as less than 30 micrometers, for example less than 25 micrometers.

D90 means that 90% of the volume of particles have a diameter less than a specified diameter. Thus, a D90 of 25 micrometers means that 90% of the volume of particles in an apixaban composition have a diameter less than 25 micrometers, as measured by laser light scattering.

In some embodiments of the composition of the invention, the composition comprises between 3 and 3.2%, such as 3.1% of crystalline apixaban particles and said particles have a D90 of less than 25 micrometers.

The dry pellet composition of the invention comprises fillers and binders. The composition may comprise:
(i) lactose and microcrystalline cellulose in a % ratio (weight ratio) of between 99 to 1 and 90 to 10, or
(ii) mannitol and microcrystalline cellulose in a % ratio of between 99 to 1 and 90 to 10.

In some embodiments, the composition comprises lactose and microcrystalline cellulose in a % ratio of between 99 to 1 and 90 to 10, such as between 96 to 4 and 94 to 6, for example 95 to 5.

Preferably, the composition comprises between 80 and 99% of lactose, such as between 83 and 89% of lactose, for example between 85 and 86% of lactose.

Preferably, the composition of the invention comprises between 3 and 10% of microcrystalline cellulose, such as between 4 and 8% of microcrystalline cellulose, for example between 4 and 5% of microcrystalline cellulose.

The dry pellet composition of the invention comprises one or more further binders. The further binder may be povidone, also termed polyvidone or polyvinylpyrrolidone (PVP). In another embodiment, the further binder is hydroxypropyl cellulose.

The composition may comprise between 3 and 15 % of the further binder, preferably between 4 and 10% of the further binder, such as between 4 and 6% of the further binder.

In some embodiment, the composition comprises between 3 and 15 % of povidone, preferably between 4 and 10% povidone, such as between 4 and 6% povidone, for example between 4 and 5% povidone.

The dry pellet composition of the invention further comprises a surfactant.

In some embodiments, the composition comprises between 0.5 and 8% of surfactant, preferably between 1 and 5% of surfactant, such between 1 and 3% of surfactant.

The surfactant may be sodium lauryl sulfate. In some embodiments, the composition comprises between 0.5 and 8% sodium lauryl sulfate, preferably between 1 and 5% sodium lauryl sulfate, such between 1 and 3% sodium lauryl sulfate.

In some embodiments, the dry composition of the invention comprises:
(i) between 3 and 3.5% of crystalline apixaban particles,
(ii) between 83 and 89% of lactose and between 4 and 5% of microcrystalline cellulose,
(iii) between 4 and 6% of povidone, and
(iv) between 1 and 3% of sodium lauryl sulfate.

Most preferably, the dry composition of the invention comprises:
(i) between 3 and 3.2% of crystalline apixaban particles having a D90 of less than 25 micrometers, as measured by laser light scattering,
(ii) between 85 and 86% of lactose and between 4 and 5% of microcrystalline cellulose,
(iii) between 4 and 5% of povidone, and
(iv) between 1 and 3% of sodium lauryl sulfate.

The pellets of the composition of the invention are typically not coated, i.e. do not comprise a core and surface coating comprising apixaban. Apixaban is typically dispersed throughout the pellets.

A dry composition according to the invention may have equivalent pharmacokinetics in human subjects compared to a tablet composition comprising the same amount of apixaban.

Thus, a dry composition according to the invention comprising 5 mg of apixaban may have equivalent pharmacokinetics in human subjects compared to an Eliquis^{®} tablet composition comprising 5 mg (https://www.ema.europa.eu/en/documents/product-information/eliquis-epar-product-information_en.pdf).

Thus, the composition of the invention may yield a Cmax (maximum concentration in plasma) that has a 90% confidence interval between 80% and 125% of that of a tablet composition, such as an Eliquis^{®} tablet composition.

Alternatively, or in addition, the composition of the invention may yield an AUC (area under the concentration-time curve) has a 90% confidence interval between 80% and 125% of that of a tablet composition, such as an Eliquis^{®} tablet composition.

In a further aspect, the invention relates to a dose-unit container comprising a dry pellet composition according to the invention as described herein.

The dose-unit container may be any suitable type of container for pellets. Preferably, the dose unit container is a capsule, such as a capsule made of gelatin or HPMC. In other embodiments, the dose unit container is a sachet.

In some embodiments, the dose-unit container contains up to 5 mg of apixaban, such as from 2.5 mg to 5 mg of apixaban.

The invention further relates to a dry composition or dose-unit container according to the invention for use as a medicament.

The invention further relates to a dry composition or dose-unit container according to the invention for use in the prevention and treatment of thromboembolic disorders.

The invention further relates to a dry composition or dose-unit container according to the invention for use in the prevention and treatment of thromboembolic disorders in an adult patient, such as a patient over 18 years of age or an elderly patient over 65 years of age, or a patient having difficulties to swallow a tablet.

In one embodiment, the composition is for use in the prevention of venous thromboembolic events (VTE) in adult patients who have undergone elective hip or knee replacement surgery.

In one embodiment, the composition is for use in the prevention of stroke and systemic embolism in adult patients with non-valvular atrial fibrillation (NVAF).

In one embodiment, the composition is for use in the treatment of deep vein thrombosis (DVT) and pulmonary embolism (PE) and prevention of recurrent DVT and PE in adults.

The composition may be administered via any suitable route of administration, including in particular oral or nasogastric administration.

As described above, in a further main aspect, the invention relates to a method for the preparation of a dry pellet composition comprising apixaban, comprising the steps of:
(i) Mixing excipients and apixaban simultaneously or sequentially,
(ii) Adding an amount of an aqueous liquid sufficient to obtain a paste,
(iii) Extruding, spheronising and drying to obtain dry pellets, optionally comprising a further step of selecting pellets having a size within a pre-determined size range.

The excipients may comprise or consist of lactose, microcrystalline cellulose, a further binder which is not microcrystalline cellulose, and surfactant.

In one embodiment, step (i) comprises mixing:
(i) between 3 and 3.5% of crystalline apixaban particles,
(ii) between 83 and 89% of lactose and between 4 and 5% of microcrystalline cellulose,
(iii) between 4 and 6% of povidone, and
(iv) between 1 and 3% of sodium lauryl sulfate.

Preferably, step (i) comprises mixing:
(i) crystalline apixaban particles having a D90 of less than 50 micrometers, as measured by laser light scattering,
(ii) lactose and microcrystalline cellulose in a % ratio of between 99 to 1 and 90 to 10 or mannitol and microcrystalline cellulose in a % ratio of between 99 to 1 and 90 to 10,
(iii) a further binder which is not microcrystalline cellulose, and
(iv) a surfactant,

More preferably, step (i) comprises mixing:
(i) between 3 and 3.5% of crystalline apixaban particles, crystalline apixaban particles having a D90 of less than 25 micrometers
(ii) between 83 and 89% of lactose and between 4 and 5% of microcrystalline cellulose,
(iii) between 4 and 6% of povidone, and
(iv) between 1 and 3% of sodium lauryl sulfate.

Mixing may be performed in a planetary mixer or a high shear mixer. In one embodiment, all ingredients are mixed simultaneously in step (i). In another embodiment, the ingredients are added and mixed sequentially. In one embodiment, the excipients are mixed first and then apixaban is added and mixed with the excipients. In such an embodiment, the second step of mixing with apixaban may be performed by "sandwich mixing", i.e. adding half of the excipient mix, then apixaban particles and then the other half of the excipient mix.

The aqueous liquid added in step (ii) may be water.

In a further aspect, the invention relates to a dry pellet composition obtainable by the method according to the invention.

Preferably, the invention relates to a dry pellet composition obtainable by the method according to the invention, comprising:
(i) between 3 and 3.5% of crystalline apixaban particles, having a D90 of less than 25 micrometers,
(ii) between 83 and 89% of lactose and between 4 and 5% of microcrystalline cellulose,
(iii) between 4 and 6% of povidone, and
(iv) between 1 and 3% of sodium lauryl sulfate.

### EXAMPLES

### Example 1 - Manufacturing process

The following process was used for producing pellet formulations to be tested:
i) Mixing of the excipients in a planetary mixer,
ii) Addition of apixaban and further mixing in a planetary mixer,
iii) Addition of water and further mixing in a planetary mixer until a paste is obtained,
iv) Extrusion and spheronization in a Caleva extruder/spheronizer (extruder: mesh size 1 mm / speed 5016 rpm; spheronizer 450145 rpm,
v) Drying in a drying oven at 60°C,
vi) Sieving to obtain pellets between 800 and 1600 micrometers.

### Example 2 - Formulations tested

A summary of the pellet formulations that were investigated during these studies is presented here below.

**Table 1:**

| **Formulation components** | **APX 001** | **APX 002** | **APX 003** | **APX 004** | **APX 005** | **APX 006** | **APX 007** | **APX 008** | **APX 009** | **APX 010** |
|---|---|---|---|---|---|---|---|---|---|---|
| **Batch size (units)** | **2500** | **2500** | **2500** | **2500** | **2500** | **2500** | **2500** | **2500** | **2500** | **2500** |
| Apixaban micronized grade (mg) | 5.0 | 5.0 | 5.0 | 5.0 | 5.0 | 5.0 | 5.0 | 5.0 | 5.0 | |
| Apixaban milled grade (mg) | | | | | | | | | | 5.0 |
| Lactose monohydrate (mg) | 108.2 | 129.9 | 137.1 | | | 133.3 | 129.9 | 140.0 | 134.1 | 137.1 |
| Mannitol (mg) | | | | 108.2 | 129.9 | | | | | |
| Cellulose microcrysta lline (mg) | 36.1 | 14.4 | 7.2 | 36.1 | 14.4 | 14.8 | 6.8 | 7.4 | 7.1 | 7.2 |
| Povidone K29/32 (mg) | 7.6 | 7.6 | 7.6 | 7.6 | 7.6 | | 15.2 | 7.6 | 7.6 | 7.6 |
| HPC (mg) | | | | | | 3.9 | | | | |
| Sodium Lauryl Sulfate (mg) | 3.1 | 3.1 | 3.1 | 3.1 | 3.1 | 3.1 | 3.1 | | 6.2 | 3.1 |
| Total (mg) | 160.0 | 160.0 | 160.0 | 160.0 | 160.0 | 160.0 | 160.0 | 160.0 | 160.0 | 160.0 |

### Example 3 - Fillers

The influence of type and content of filler was tested, by testing the dissolution profiles of formulations comprising microcrystalline cellulose and lactose or mannitol. Microcrystalline cellulose (MCC) serves as filler but also as a binder. Lactose serves as filler. Mannitol was studied as an alternative to lactose.

Compositions APX001 (lactose/MCC ratio 75/25), APX002 lactose/MCC ratio 90/10), APX003 lactose/MCC ratio 95/5), APX004 mannitol/MCC ratio 75/25) and APX005 mannitol/MCC ratio 90/10) described in Example 2 were tested.

Dissolution tests were performed in 900 mL of 0.01N HCl at 37°C, using Ph. Eur apparatus type 2 (paddles) method at a rotation speed of 75 rpm. Samples were removed after 5, 10, 15, 20, 30, 45, 60 and 90 min from test initiation and analyzed for apixaban by HPLC. Dissolution tests were carried out on a coni-snap gelatin size T2 capsule filled with 160 mg of pellets (5 mg apixaban).

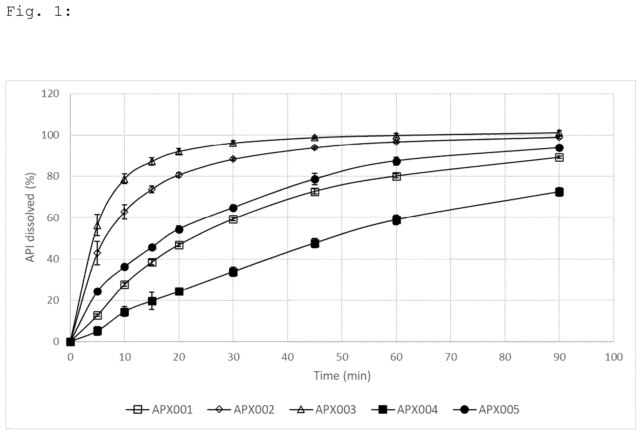

Data are shown in Figure 1. It can be seen that increasing the microcrystalline cellulose content slows down the dissolution of apixaban. Furthermore, using mannitol instead of lactose also somewhat slows down the dissolution of apixaban.

### Example 4 - Binders

Without the addition of binder, no granulation of the material was obtained. As candidate binders, two different concentrations of povidone (PVP) were tested: 4.7% (w/w) (APX003) and a higher 9.5% (w/w) (APX007). The use of a PVP concentration below 4.7% did not result in the formation of pellets but rather in a fine powder.

Dissolution test were performed in 900 mL of 0.01N HCl at 37°C, using Ph. Eur apparatus type 2 (paddles) method at a rotation speed of 75 rpm. Samples were removed after 5, 10, 15, 20, 30, 45, 60 and 90 min from test initiation and analyzed for apixaban by HPLC. Dissolution tests were carried out on a coni-snap gelatin size T2 capsule filled with 160 mg of pellets (5 mg apixaban).

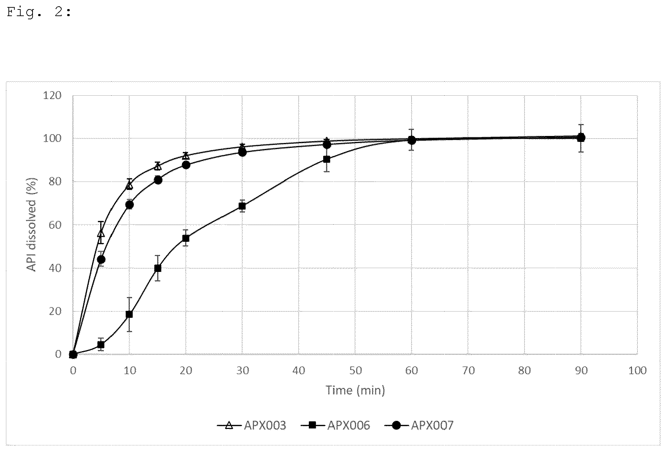

Figure 2 shows that a higher content of povidone (9.5% w/w) had no influence on the dissolution profile of apixaban.

Using hydroxypropyl cellulose at a concentration of 4.7% led to the formation of agglomerates that were too large, prompting a test with a lower concentration. At 2.25% (APX006), the resulting pellets were satisfactory, but the dissolution profile was slower than when povidone was used (Figure 2).

### Example 5 - Surfactant

Three different levels of the surfactant sodium lauryl sulfate were tested: 0% (w/w) (APX008), 1.9% (w/w) (APX003) and 3.8% (APX009).

Dissolution test were performed in 900 mL of 0.01N HCl at 37°C, using Ph. Eur apparatus type 2 (paddles) method at a rotation speed of 75 rpm. Samples were removed after 5, 10, 15, 20, 30, 45, 60 and 90 min from test initiation and analyzed for apixaban by HPLC. Dissolution tests were carried out on a coni-snap gelatin size T2 capsule filled with 160 mg of pellets (5 mg apixaban).

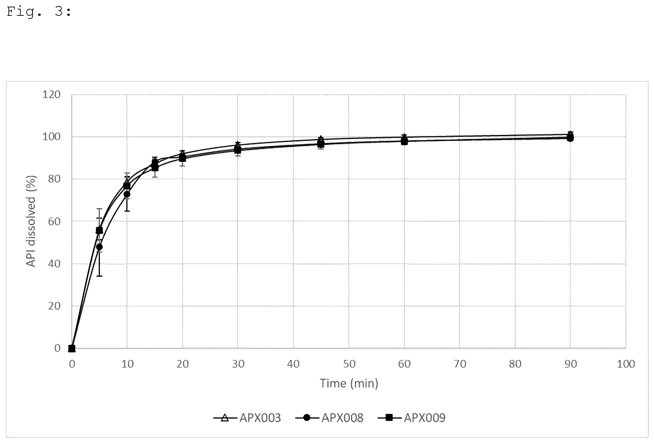

Figure 3 shows that the sodium lauryl sulfate content did not influence dissolution profile of apixaban between 0 and 3.8%.

### Example 6 - Particle size

To test the possible impact of particle size, we tested two grade offers from the supplier: a micronized grade (Dv0.9 < 25 um) (APX003) and a milled grade (Dv0.9 > 90 um) (APX010).

Dissolution tests using apparatus type 2 (paddle) were carried out on a coni-snap gelatin size T2 capsule filled with 160 mg of pellets (5 mg apixaban).

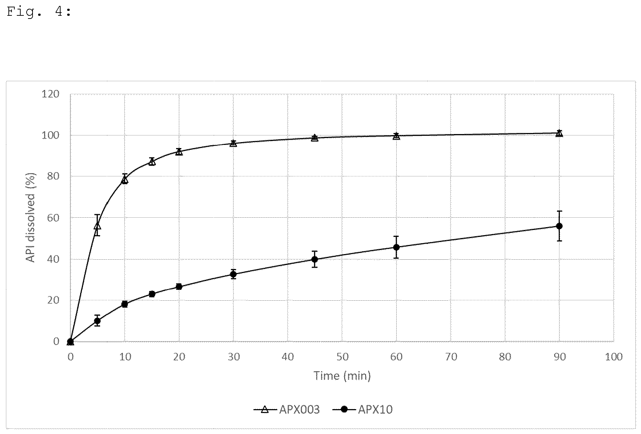

Figure 4 shows that large particle size of apixaban negatively impacted its immediate release dissolution profile.

### Example 7 - Pharmacokinetic study

A bioequivalence comparison was performed between a APX003 formulation (pellets in capsule) of apixaban 5 mg and a marketed formulation (Eliquis^{®} 5 mg). A single oral dose was administered in fasting conditions to 36 healthy participants (two-treatment, two-period, two-sequence, open, crossover, randomized with at least 5 days wash-out).

A total of 72 participants were screened and 36 of them were randomised in the study. Thirty-four (34) participants completed the study and were included in the statistical analysis.

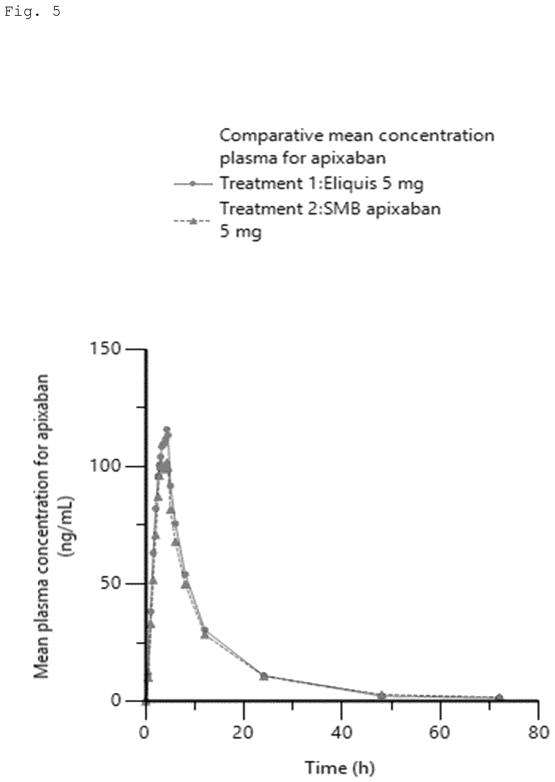

Plasma concentrations of apixaban over time were compared. The data are show in Figure 5 and Table 2.

**Table 2: GMRs are expressed in % of the Eliquis^{®} value, CI 90% are the confidence interval ranges of these GMR values**

| Pharmacokinetic metrics | Geometric mean ratio (GMR) | CI 90% Lower | CI 90% Upper |
|---|---|---|---|
| AUC- (ng/mL.h) | 93.07 | 89.38 | 96.91 |
| AUCt (ng/mL.h) | 92.20 | 88.56 | 95. 98 |
| Cₘₐₓ (ng/mL) | 86.72 | 81.41 | 92.38 |

The results demonstrated a comparable rate and extent of exposure for apixaban between APX003 5 mg and Eliquis^{®} 5 mg.

## Claims

1. Dry composition in the form of pellets suitable for facilitating administration of apixaban, comprising:
(i) crystalline apixaban particles having a D90 of less than 50 micrometers, as measured by laser light scattering,
(ii) lactose and microcrystalline cellulose in a % ratio of between 99 to 1 and 90 to 10, or
mannitol and microcrystalline cellulose in a % ratio of between 99 to 1 and 90 to 10,
(iii) a further binder which is not microcrystalline cellulose, and
(iv) a surfactant.

2. Dry composition according to claim 1, wherein the pellets have a diameter of between 200 micrometers and 2000 micrometers, such as between 800 micrometers and 1600 micrometers.

3. Dry composition according to any one of the preceding claims, wherein the composition comprises between 1 and 5% (w/w) of crystalline apixaban particles, such as between 3 and 3.5% of crystalline apixaban particles.

4. Dry composition according to any one of the preceding claims, wherein the crystalline apixaban particles have a D90 of less than 40 micrometers, such as less than 30 micrometers, for example less than 25 micrometers.

5. Dry composition according to any one of the preceding claims, wherein the composition comprises lactose and microcrystalline cellulose in a % ratio of between 99 to 1 and 90 to 10, wherein the composition comprises between 80 and 95% of lactose, such as between 83 and 89% of lactose, and wherein the composition comprises between 3 and 10% of microcrystalline cellulose, preferably between 4 and 8% of microcrystalline cellulose, such as between 4 and 5% of microcrystalline cellulose.

6. Dry composition according to any one of the preceding claims, wherein the further binder is povidone.

7. Dry composition according to any one of the preceding claims, wherein the composition comprises between 3 and 15 % of the further binder, preferably between 4 and 10% of the further binder, such as between 4 and 6% of the further binder.

8. Dry composition according to any one of the preceding claims, wherein the composition comprises between 0.5 and 8% of surfactant, preferably between 1 and 5% of surfactant, such between 1 and 3% of surfactant and wherein the surfactant preferably is sodium laurylsulfate.

9. Dry composition according to any one of the preceding claims, wherein the composition comprises
(i) between 3 and 3.5% of crystalline apixaban particles,
(ii) between 83 and 89% of lactose and between 4 and 5% of microcrystalline cellulose,
(iii) between 4 and 6% of povidone, and
(iv) between 1 and 3% of sodium laurylsulfate.

10. Dose-unit container comprising a dry pellet composition according to any one of the preceding claims, wherein the dose-unit container preferably is a capsule and/or wherein the dose-unit container contains up to 5 mg of apixaban, such as from 2.5 mg to 5 mg of apixaban.

11. Dry composition or dose-unit container according to any one of the preceding claims for oral or nasogastric administration.

12. Dry composition or dose-unit container according to any one of the preceding claims for use as a medicament, preferably for use in the prevention and treatment of thromboembolic disorders.

13. A method for the preparation of a dry pellet composition comprising apixaban, comprising the steps of:
(i) Mixing excipients and apixaban simultaneously or sequentially,
(ii) Adding an amount of an aqueous liquid sufficient to obtain a paste,
(iii) Extruding, spheronising and drying to obtain dry pellets, optionally comprising a further step of selecting pellets having a size within a pre-determined size range.

14. The method according to claim 13, wherein step (i) comprises mixing:
(i) crystalline apixaban particles having a D90 of less than 50 micrometers, as measured by laser light scattering,
(ii) lactose and microcrystalline cellulose in a % ratio of between 99 to 1 and 90 to 10 or mannitol and microcrystalline cellulose in a % ratio of between 99 to 1 and 90 to 10,
(iii) a further binder which is not microcrystalline cellulose, and
(iv) a surfactant,
wherein, preferably, step (i) comprises mixing:
(i) between 3 and 3.5% of crystalline apixaban particles, having a D90 of less than 25 micrometers,
(ii) between 83 and 89% of lactose and between 4 and 5% of microcrystalline cellulose,
(iii) between 4 and 6% of povidone, and
(iv) between 1 and 3% of sodium laurylsulfate.

15. A dry pellet composition obtainable by the method according to claim 13 or 14.
